# EUROPÄISCHE PATENTANMELDUNG

(11) **EP 1 099 385 A2**
(43) Veröffentlichungstag der Anmeldung: **16.05.2001**
(21) Anmeldenummer: 00123375.8
(22) Anmeldetag: 31.10.2000
(51) Int. Cl.: A23L 1/22

(54) **Eingekapseltes Aroma**

(30) Priorität: 12.11.1999 DE 19954528
(71) Anmelder: HAARMANN & REIMER GMBH, 37601 Holzminden (DE)
(72) Erfinder: Schleifenbaum, Birgit, 37671 Höxter (DE); Uhlemann, Jens, Dr., 37603 Holzminden (DE); Renz, Karl-Heinz, 37603 Holzminden (DE)
(74) Vertreter: Mann, Volker, Dr.

(57) **Zusammenfassung**

In Kohlenhydrate eingekapselte Aromen, deren Oberfläche mit einem inerten Gas behandelt wurde, werden bevorzugt zum Aromatisieren von Lebensmittelprodukten, Bedarfsgegenständen und von Pharmazeutika verwendet.

## Beschreibung

Die Erfindung betrifft aromahaltige Partikel, deren Oberfläche mit einem inerten Gas behandelt wurde. Die so erhaltenen eingekapselten Aromen werden bevorzugt zum Aromatisieren von Lebensmittelprodukten und von Pharmazeutika verwendet.

Eingekapselte Aromen mit einer besonders guten Lagerstabilität werden in der Aromenindustrie in der Regel durch Emulgierung des Aromas in geschmolzenen Kohlenhydratmischungen mit anschließender Formgebung hergestellt. Hierbei wird die Kohlenhydratschmelze mit emulgiertem Aroma beispielsweise durch eine Lochplatte in ein vorgelegtes, gekühltes Isopropanolbad entspannt (US 4 707 367, US 4 499 112, US 3 704 137, US 3 041 180 und US 2 809 895). In dem Isopropanolbad werden die Stränge beim Erstarren von einem Rührer auf Korngrößen zwischen etwa 0,3 und 1,5 mm zerkleinert. Die dabei entstehenden Partikel werden in diesem Lösungsmittelbad gleichzeitig von Aromaanteilen, die der Partikel-Oberfläche anhaften, zu einem großen Teil befreit. Das eingekapselte Aroma wird anschließend durch Zentrifugieren und schonende Vakuumtrocknung getrocknet.

Nachteile dieser Verfahren liegen in dem aufwendigen Lösungsmittel-Handling begründet.

Die bekannten Partikel weisen eine niedrige Glasübergangstemperatur auf und verklumpen daher leicht.

Aus dem zum Waschen verwendeten Lösungsmittel müssen für eine Rückführung durch Feinfiltration die Zuckerreste und durch Destillation die Wasser- und Aromenreste entfernt werden. Trotz dieser Aufarbeitung müssen kontaminierte Lösungsmittel in getrennten Tanks zwischengelagert werden. Das gesamte Herstellungsverfahren muss einen hohen Sicherheitsstandard erfüllen. Die Anlage muss explosionsgeschützt ausgeführt werden und die Flexibilität bezüglich Produktwechsel ist wegen der Kontaminationsproblematik sehr eingeschränkt.

Durch Verwendung eines Extruders und Erstarrung an der Luft können prinzipiell ausgeformte Stränge auch ohne Einsatz eines Lösungsmittels hergestellt werden. Diese Verfahrensweise ist bekannt (US 5 603 971, US 5 601 865, US 5 087 461, US 5 786 017, WO 94/06308, WO 94/23593, US 5 009 900 und US 4 820 534). Die Kühlung kann durch ein Kühlband oder ähnliches erfolgen. Die Zerkleinerung muss in einem nachgeschalteten mechanischen Zerkleinerungsverfahren (z.B. Schneidmühle oder Brecher) durchgeführt werden. Bei der Zerkleinerung auf die gewünschte Korngröße zwischen etwa 0,3 und 1,5 mm wird die Oberflächenstruktur der Partikel zerstört. Damit ist nachteilig eine erhöhte Beladung mit Oberflächenaromen verbunden. Außerdem bildet sich ein unerwünscht hoher Anteil von Feinstaub.

Die an der Oberfläche befindliche ölige Schicht aus dem eingesetzten Aroma bewirkt eine starke Verschlechterung der Lagerstabilität der Partikel.

Es werden also in Kohlenhydrate eingekapselte Aromen gesucht, deren Oberfläche frei von einer öligen Schicht aus dem eingesetzten Aroma ist, und die eine hohe Glasübergangstemperatur aufweisen. Das Herstellungsverfahren soll
- 1.: eine kontinuierliche Ausführung der Herstellung,
- 2.: die Integration der Einzelschritte Ausformung, Abkühlung und Zerkleinerung in das Herstellungsverfahren,
- 3.: ein lösungsmittelfreies Verfahren zur Verringerung des Oberflächenaromas und
- 4.: eine integrierte Entstaubung

### ermöglichen.

Es wurden in Kohlenhydrate eingekapselte Aromapartikel, hergestellt durch Emulgieren des Aromas in eine Kohlenhydratschmelze und Herstellung der Partikeln aus der so erhaltenen Schmelze, gefunden, dadurch gekennzeichnet, dass die Partikel mit inerten Gasen behandelt werden.

Die erfindungsgemäßen neuen Aromapartikel sind an der Oberfläche praktisch frei von einer öligen Schicht aus dem eingesetzten Aroma und weisen eine hohe Glasübergangstemperatur auf.

Das Aroma befindet sich praktisch ausschließlich im Innern der Partikel.

Kohlenhydrate für die erfindungsgemäß eingekapselten Aromen sind bevorzugt hydrolysierte Stärken, Mono- und/oder Disaccharide, wie Maltose.

Die Aromapartikel weisen eine zylindrische oder kugelige Geometrie und ein enges Korngrößenprofil auf. Sie haben einen Durchmesser von 0,3 bis 12 mm, bevorzugt 0,5 bis 1,0 mm, und eine Länge von 0,3 bis 10 mm, bevorzugt von 0,5 bis 1 mm.

Die erfindungsgemäßen Partikel haben einen Aromagehalt von 1 bis 25 Gew.-%, bevorzugt von 3 bis 10 Gew.-%.

Die erfindungsgemäßen Partikel haben eine Glasübergangstemperatur im Bereich von 45 bis 75°C, bevorzugt von 50 bis 60°C (DSC Methode, Aufheizrate 20K/min).

Es ist selbstverständlich möglich, dass die erfindungsgemäßen Aromapartikel noch weitere Stoffe, wie z.B. Emulgatoren, Farbstoffe und andere Füllstoffe enthalten.

Die erfindungsgemäßen Aromapartikel können zur Aromatisierung von Lebensmittelprodukten, wie beispielsweise Instantgetränkepulvern, Tee, Suppen- oder Soßenpulvern, Süßwaren, Kaugummi, sowie von Pharmazeutika und auch von Bedarfsgegenständen angewendet werden.

Die erfindungsgemäßen Aromapartikel können auch zum Aromatisieren von Bedarfsartikeln, wie Mundhygieneprodukten (z.B. Gebissreinigungstabletten und Zahnpasten), Kosmetik-, Seifen-, Hygiene-, Detergenzien oder Haushaltsprodukten angewendet werden.

Die erfindungsgemäßen Aromapartikel können auch zum Aromatisieren von Pharmazeutika, wie Lutschtabletten, Kautabletten, Tabletten zum Auflösen in Wasser, Lutschbonbons, Kaubonbons, Aufgussformulierungen angewendet werden.

Selbstverständlich ist es möglich, anstelle von Aromen Riechstoffe erfindungsgemäß einzusetzen.

Es wurde auch ein Verfahren zur Herstellung von in Kohlenhydrate eingekapselte Aromen, hergestellt durch Emulgieren des Aromas in eine Kohlenhydratschmelze und Herstellung von Partikeln aus der so erhaltenen Schmelze, gefunden, das dadurch gekennzeichnet ist, dass die Partikel mit inerten Gasen behandelt werden.

Inerte Gase für das erfindungsgemäße Verfahren sind Stickstoff, Edelgase wie Helium und Argon, und Luft. Bevorzugtes inertes Gas ist Luft.

Das erfindungsgemäße Verfahren wird im allgemeinen im Temperaturbereich von 10 bis 35°C, bevorzugt von 20 bis 25°C, durchgeführt.

Die Behandlung der Partikel mit dem Gasstrom erfolgt durch Durchleiten des inerten Gases durch einen konvektiven Trockner mit einer Gasgeschwindigkeit von 0,2 bis 4 m/s, bevorzugt von 0,5 bis 2 m/s.

Konvektive Trockner, z.B Wirbelschichtapparate, sind an sich bekannt.

Das erfindungsgemäße Verfahren kann sowohl diskontinuierlich als auch kontinuierlich durchgeführt werden.

Beispielsweise kann das erfindungsgemäße Verfahren in den folgenden Stufen durchgeführt werden:

Herstellung der Schmelze

Die Herstellung erfolgt in den Einzelschritten:
a. Trockenmischung aufschmelzen
b. Aroma in die Trockenmischung emulgieren

- Eine geeignete trockene Kohlenhydratmischung wird hergestellt und durch Erhitzen auf 80 bis 120°C, bevorzugt auf 90 bis 100°C, in einem Extruder aufgeschmolzen. Vorzugsweise wird dazu ein Doppelwellen-Extruder mit mehreren Temperaturzonen verwendet.
- Das Aroma, das zusätzlich einen geeigneten Emulgator enthalten kann, wird mit einer Dosierung von vorzugsweise 1 bis 25, bevorzugt 3 bis 10, Gew.-% bezogen auf die Trockenmischung über eine Pumpe kontinuierlich in den vorderen Extruderbereich gegeben. Die Extruderwerkzeuge sorgen für die Emulgierung des Aromas in der Schmelze.

### Integration der Einzelschritte Ausformung, Abkühlung und Zerkleinerung bei der Herstellung der Partikel

Zur Ausformung der Stränge nach der Lochplatte muß die Schmelze gekühlt werden. Bevorzugt wird dies durch konzentrisches Anblasen der Lochplatte mit Kaltluft realisiert. Dabei muß auf die gleichmäßige Temperierung der Lochplatte geachtet werden. Die Zerkleinerung der Stränge erfolgt noch in der Erstarrungsphase durch eine Kopfgranulierung. Dazu wird bevorzugt eine gasdichte Ausführung der Kopfgranulierung mit rotierenden Messern verwendet. Eine stufenlose Drehzahlregelung der Kopfgranulierung ermöglicht die Einstellung der Partikellänge in Abhängigkeit vom Feststoff-Durchsatz. Die dabei erhaltenen Partikel weisen ein Schüttgewicht von ca. 0,5 kg/l auf.

### Verfahren zur Entfernung des Oberflächenaromas

Bei diesem Verfahrensschritt erfolgt die erfindungsgemäße Behandlung der Partikel mit einem inerten Gas.

Die Kontaktierung kann in konvektiven Trocknern durch Anströmen, Verwirbeln oder Durchmischen bewirkt werden. Bei einer Wirbelschichtapparatur wird hierbei ein Gasdurchsatz benötigt, der einer Leerrohrgeschwindigkeit von 0,2 bis 4 m/s, bevorzugt 0,5 bis 2 m/s, entspricht und eine Durchströmzeit im Bereich von 5 bis 120 min, bevorzugt von 20 bis 40 min, bei einer Füllhöhe von 0,01 bis 0,5 m, bevorzugt von 0,05 bis 0,2 m.

### Integrierte Entstaubung

Beim Kontaktieren der Partikel mit inertem Gas wird anhaftender oder neu entstehender Feinstaub vom Gasstrom mitgerissen. Das staubbeladene Abgas kann über eine geeignete Entstaubungsanlage geführt werden, so dass eine nachgeschaltete Siebung der Partikel entfällt.

Hinter den konvektiven Trockner kann ein sichtender Produktaustrag (z.B. Zick-Zack-Sichter, Steigrohrsichter o.ä.) geschaltet werden.

Durch das erfindungsgemäße Verfahren erhält man Aromapartikel mit einer geringen Oberflächenaromabeladung durch das Aroma und einer hohen Glasübergangstemperatur.

### Beispiele:

### Herstellungsbeispiele

### Beispiel 1 Herstellung von Zitronenaromapartikeln

Zitronenaroma wird zu 5 % in eine Schmelze von verschiedenen Maltodextrinen, Disacchariden und einem Emulgator im Extruder eingearbeitet. Über eine 0,5 mm Lochplatte werden Stränge geformt, die mittels Kopfgranulation auf eine Länge von 0,5 bis 1 mm zerkleinert werden. Anschließend wird 1 kg der Partikel in einer diskontinuierlich betriebenen Wirbelschichtapparatur 60 Minuten lang mit Luft kontaktiert. Zur Fluidisierung des Bettinhaltes wird Luft mit einer Leerohrgeschwindigkeit von 1,25 m/s eingeblasen. Die Eintrittstemperatur des Fluidisiergases beträgt 25°C. Die Temperatur des Abgases beträgt 25°C. Anschließend werden Staubanteile über ein 0,5 mm Sieb entfernt.

### Anwendungsbeispiele

### Beispiel 2 Instantgetränkepulver

Aromatisierung einer Instantgetränke - Pulvermischung bestehend aus 90 Gew.-% Saccharose, 8 Gew.-% Zitronensäure, 1 Gew.-% weiteren Zutaten (Calcium-Phosphat, Ascorbinsäure, modifizierter Cellulose, Farbstoff ) und 1 Gew.-% gelb gefärbten Zitronenaromapartikeln (Ø 0,4-0,6 mm), die nach der beschriebenen Verfahrensweise hergestellt wurden. Die Mischung zeichnet sich durch eine besonders gute Lagerstabilität des Aromas auch bei geringer Korngröße aus. Aufgrund des geringen Anteils des oxidationsempfindlichen Zitronenaromas an der Oberfläche der Partikel ist die Entstehung von Fehlnoten (bedingt durch Oxidation) sehr stark minimiert.

### Beispiel 3 Beuteltee

Aromatisierung von schwarzem Tee in Teebeuteln mit 3 Gew.-% Erdbeeraromapartikeln (Ø 1 mm, Länge 1 - 2 mm). Während der Lagerung des Tees bleibt das Aroma in der Granulatmatrix eingeschlossen, und wird erst beim Aufbrühen durch Lösen der Partikelmatrix in heißem Wasser freigesetzt.

### Beispiel 4 Kaugummi

Kaugummimasse wird mit blau gefärbten Pfefferminzaromapartikeln (Ø 0,6 mm, Länge 0,4 mm), die nach dem beschriebenen Verfahren hergestellt wurden, versetzt. Die Partikel erzeugen einen besonderen optischen Effekt. Die Freisetzung des Aromas erfolgt mechanisch beim Kauen.

### Beispiel 5 (Vergleich)

In der folgenden Gegenüberstellung (Tabelle 1) werden die erfindungsgemäßen Aromapartikel vor und nach der Kontaktierung mit Luft verglichen. Es zeigt sich, dass mit dem erfindungsgemäßen Verfahren eine lösungsmittelfreie Herabsetzung des Oberflächenaromas möglich ist. Bestimmung des Oberflächenaromas: Waschen der Partikel mit Pentan/Ether (Partikel werden dabei nicht aufgelöst), Analyse des Waschwassers auf Gehalt an Aroma (GC), Ergebnis wird dann bezogen auf Einwaage Granulat, Angabe in ppm.

**Tabelle 1:**

| | Vor der Luftbehandlung | Nach der Luftbehandlung (Wirbelbett 40 min, 1,25 m/s, 25°C (erfindungsgemäß) |
|---|---|---|
| Citronen-Granulat (Ø 1 mm) | 197 ppm | 9 ppm |
| Erdbeer-Granulat (Ø 1 mm) | 943 ppm | 4 ppm |

## Patentansprüche

1. In Kohlenhydrate eingekapselte Aromapartikel, hergestellt durch Emulgieren des Aromas in eine Kohlenhydratschmelze und Herstellung der Partikeln aus der so erhaltenen Schmelze, dadurch gekennzeichnet, dass die Partikel mit inerten Gasen behandelt werden.

2. Eingekapselte Aromen nach Anspruch 1, dadurch gekennzeichnet, dass sie eine Glasübergangstemperatur von 45 bis 75°C aufweisen.

3. Eingekapselte Aromen nach den Ansprüchen 1 und 2, dadurch gekennzeichnet, dass sie einen Durchmesser im Bereich von 0,3 mm bis 12 mm und eine Länge von 0,3 bis 10 mm aufweisen.

4. Eingekapselte Aromen nach den Ansprüchen 1 bis 3, dadurch gekennzeichnet, dass sie einen Aromagehalt von 1 bis 25 Gew.-% aufweisen.

5. Eingekapselte Aromen nach den Ansprüchen 1 bis 4, dadurch gekennzeichnet, dass sie als Kohlenhydrat hydrolysierte Stärke, Mono-und/oder Disaccharide enthalten.

6. Eingekapselte Aromen nach den Ansprüchen 1 bis 5, dadurch gekennzeichnet, dass sie Aromen, natürliche Extrakte, Nutraceuticals und/oder Lebensmittelzusatzstoffe, enthalten .

7. Lebensmittel, enthaltend in Kohlenhydrate eingekapselte Aromen nach den Ansprüchen 1 bis 6.

8. Pharmazeutika, enthaltend in Kohlenhydrate eingekapselte Aromen nach den Ansprüchen 1 bis 6.

9. Bedarfsgegenstände, enthaltend in Kohlenhydrate eingekapselte Aromen nach den Ansprüchen 1 bis 6.

10. Verwendung von eingekapselten Aromen nach den Ansprüchen 1 bis 6 zum Aromatisieren von Lebensmitteln.

11. Verwendung von eingekapselten Aromen nach den Ansprüchen 1 bis 6 zum Aromatisieren von Pharmazeutika.

12. Verwendung von eingekapselten Aromen nach den Ansprüchen 1 bis 6 zum Aromatisieren von Bedarfsgegenständen.

13. Verfahren zur Herstellung von eingekapselten Aromen, hergestellt durch Emulgieren des Aromas in eine Kohlenhydratschmelze und Herstellung von Partikeln aus der so erhaltenen Schmelze, dadurch gekennzeichnet, dass die Partikel mit inerten Gasen behandelt werden.

14. Verfahren nach Anspruch 13, dadurch gekennzeichnet, dass als inertes Gas Luft verwendet wird.

15. Verfahren nach den Ansprüchen 13 und 14, dadurch gekennzeichnet, dass die Behandlung mit dem inerten Gas im Temperaturbereich von 10 bis 35°C erfolgt.

16. Verfahren nach den Ansprüchen 13 bis 15, dadurch gekennzeichnet, dass die Behandlung in einem Gasstrom mit einer Gasgeschwindigkeit von 0,2 bis 4 m/s erfolgt.
